# EUROPEAN PATENT APPLICATION

(11) **EP 1 000 931 A1**
(43) Date of publication of application: **17.05.2000**
(21) Application number: 99308983.8
(22) Date of filing: 11.11.1999
(51) Int. Cl.: C07C 271/22

(54) **Process for producing an N-alkoxycarbonyl-N-cyanoethyl amino acid alkyl ester**

(30) Priority: 13.11.1998 JP 32345098
(71) Applicant: TOKUYAMA CORPORATION, Tokuyama-shi, Yamaguchi-ken 745-0053 (JP)
(72) Inventor: Hirano, Naoki, Tokuyama-shi, Yamaguchi 745-0053 (JP); Iwasaki, Fumiaki, Tokuyama-shi, Yamaguchi 745-0053 (JP)
(74) Representative: Sexton, Jane Helen

(57) **Abstract**

An N-alkoxycarbonyl-N-cyanoethyl amino acid alkyl ester is produced at a high yield by reacting a mineral acid salt of amino acid alkyl ester with acrylonitrile in an alcohol medium in the presence of an inorganic base to form an N-cyanoethyl amino acid alkyl ester and then reacting the N-cyanoethyl amino acid alkyl ester with a dialkyl dicarbonate.

## Description

The present invention relates to a process for producing an N-alkoxycarbonyl-N-cyanoethyl amino acid alkyl ester and an N-cyanoethyl amino acid alkyl ester, which are useful as pharmaceutical intermediates, at a high yield.

An N-alkoxycarbonyl-N-cyanoethyl amino acid alkyl ester and an N-cyanoethyl amino acid alkyl ester are extremely important compounds as precursors of pyrrolidine derivatives which are important intermediates of new quinolone-based synthetic antibacterial agents (see EP 0688772A1).

EP 0688772A1 discloses a process for producing an N-alkoxycarbonyl-N-cyanoethyl amino acid alkyl ester by causing di-t-butyl dicarbonate to act on an N-cyanoethyl amino acid alkyl ester obtained by reacting an amino acid alkyl ester hydrochloride with acrylonitrile in an aqueous solution of potassium hydroxide.

In the above process, the formed N-cyanoethyl amino acid alkyl ester is extracted with an organic solvent and then isolated by distillation. However, it is hardly said that the process is practical because the yield of the compound is as low as 48 %. Since the N-alkoxycarbonyl-N-cyanoethyl amino acid alkyl ester is produced by reacting the N-cyanoethyl amino acid alkyl ester obtained by the above process with di-t-butyl dicarbonate, its yield depends on the yield of the N-cyanoethyl amino acid alkyl ester and, hence, never exceeds 48 %. Therefore, the development of a process for obtaining an N-alkoxycarbonyl-N-cyanoethyl amino acid alkyl ester and an N-cyanoethyl amino acid alkyl ester at a high yield has been strongly desired.

It is therefore an object of the present invention to provide a process for producing an N-alkoxycarbonyl-N-cyanoethyl amino acid alkyl ester at a high yield.

It is another object of the present invention to provide a process for producing an N-cyanoethyl amino acid alkyl ester at a high yield.

Other objects and advantages of the present invention will become apparent from the following description.

According to the present invention, firstly, the above objects and advantages of the present invention are attained by a process for producing an N-alkoxycarbonyl-N-cyanoethyl amino acid alkyl ester comprising the steps of:
(1) reacting a mineral acid salt of amino acid alkyl ester with acrylonitrile in an alcohol medium in the presence of an inorganic base to form an N-cyanoethyl amino acid alkyl ester; and
(2) reacting the N-cyanoethyl amino acid alkyl ester with a dialkyl dicarbonate to form an N-alkoxycarbonyl-N-cyanoethyl amino acid alkyl ester.

According to the present invention, secondly, the above objects and advantages of the present invention are attained by a process for producing an N-cyanoethyl amino acid alkyl ester by carrying out only the above step (1).

Any known compounds having at least one amino group and at least one alkoxycarbonyl group in the molecule are used without any restriction as the amino acid alkyl ester used in the present invention. The term "amino group" as used herein refers to a group having at least one hydrogen atom bonded to a nitrogen atom, such as a so-called primary amino group (-NH₂), imino group (=NH), or secondary amino group such as monosubstituted amino group of which substituent group is a hydrocarbon group, e.g., an alkyl group or an aralkyl group, or a pyrrolidyl group. Of these, the amino group is preferably a primary amino group. As for the numbers of amino groups and alkoxycarbonyl groups, compounds having one amino group in the molecule are preferable, and compounds having one or two alkoxycarbonyl groups, preferably one alkoxycarbonyl group, in the molecule are desirable.

Illustrative examples of the amino acid alkyl ester used in the present invention include lower alkyl esters having 1 to 6 carbon atoms such as methyl, ethyl, propyl isopropyl, butyl, isobutyl, tert-butyl and the like of amino acids, as exemplified by glycine, phenylglycine, N-methylglycine, N-benzylglycine, hydroxyphenylglycine, alanine, N-methylalanine, N-ethylalanine, β-alanine, N-methyl-β-alanine, serine, homoserine, isoserine, S-acetylcysteine, cystine, threonine, methionine, homomethionine, valine, norvaline, leucine, norleucine, isoleucine, phenylalanine, hydroxyphenylalanine, tyrosine, thyronine, proline, hydroxyproline, tryptophan, aspartic acid, asparagine, glutamic acid, homoglutamic acid, glutamine, arginine, histidine, pipecolic acid, α-aminobutyric acid, β-aminobutyric acid, γ-aminobutyric acid and α-aminoisobutyric acid. Some of these amino acid alkyl esters have S-configuration and R-configuration optical isomers. Such optical isomers or racemic mixtures may be used in the present invention. Further, a mixture of different amino acid alkyl esters may be used.

A common mineral acid such as hydrochloric acid, hydrobromic acid, sulfuric acid, nitric acid or phosphoric acid may be used without any restriction as the mineral acid constituting the mineral acid salt of amino acid alkyl ester in the present invention, Of these, hydrochloric acid and sulfuric acid are particularly preferable because they facilitate the production of the mineral acid salt of amino acid alkyl ester.

A common alkyl alcohol may be used without any restriction as the alcohol medium used in the present invention. Preferable examples of the alcohol include straight-chain and branched-chain lower alkyl alcohols having 1 to 6 carbon atoms such as methanol, ethanol, propanol, isopropanol, butanol, isobutanol and tert-butanol.

Any conventionally known methods may be used in the present invention without any restriction to prepare an alcohol suspension of a mineral acid salt of amino acid alkyl ester. The methods include, for example, one in which a mineral acid salt of amino acid alkyl ester is dispersed in an alcohol, one in which an amino acid alkyl ester is dissolved in an alcohol and then neutralized with an acid in an equimolar amount to that of the amino acid alkyl ester, one in which an equimolar amount or more of a mineral acid is added to an alcohol suspension of an amino acid to carry out an esterification reaction and the obtained alcohol suspension is directly used in the present invention, one in which an esterification reaction is carried out by reacting an alcohol suspension of an amino acid with an equimolar amount or more of thionyl chloride and the obtained alcohol suspension is directly used in the present invention, and the like.

In the alcohol suspension of the mineral acid salt of the amino acid alkyl ester in the present invention, the alkyl group of the mineral acid salt of amino acid alkyl ester may be the same as or different from the alkyl group of the alcohol. However, because of a possibility that an ester interchange reaction may occur during a reaction between the mineral acid salt of amino acid alkyl ester and acrylonitrile, the present invention is preferably carried out by combining a mineral acid salt and an alcohol which have the alkyl group of the same type when the occurrence of this reaction is not desired.

A common inorganic base may be used without any restriction as the inorganic base used in the present invention. Illustrative examples of the inorganic base include alkali metal hydroxides such as sodium hydroxide, potassium hydroxide and lithium hydroxide; alkali metal carbonates such as sodium carbonate, potassium carbonate, lithium carbonate, sodium bicarbonate, potassium bicarbonate and lithium bicarbonate; alkaline earth metal hydroxides such as calcium hydroxide and magnesium hydroxide; and alkaline earth metal carbonates such as calcium carbonate, magnesium carbonate, calcium bicarbonate and magnesium bicarbonate. Of these inorganic bases, alkali metal hydroxides such as sodium hydroxide, potassium hydroxide and lithium hydroxide; and alkali metal carbonates such as sodium carbonate, potassium carbonate, lithium carbonate, sodium bicarbonate, potassium bicarbonate and lithium bicarbonate are particularly preferable because they cause a neutralization reaction to proceed easily.

When the amount of the inorganic base is too small, a mineral acid salt of amino acid alkyl ester cannot be fully neutralized and a reaction between the mineral acid salt of amino acid alkyl ester and acrylonitrile is impeded. On the other hand, when the amount is too large, the viscosity of a slurry becomes high, thereby hindering stirring. Therefore, the amount is preferably 1 to 6 mol, more preferably 1 to 4 mol, based on 1 mol of the mineral acid salt of amino acid alkyl ester.

An excess of mineral acid or sulfur dioxide is dissolved in an alcohol suspension when a method in which an esterification reaction is carried out by adding an equimolar amount or more of a mineral acid to the alcohol suspension of an amino acid and the obtained alcohol suspension is directly used in the present invention or a method in which an esterification reaction is carried out by reacting an alcohol suspension of an amino acid with an equimolar amount or more of thionyl chloride and the obtained alcohol suspension is directly used in the present invention is used to prepare an alcohol suspension of a mineral acid salt of amino acid alkyl ester. Therefore, it is desirable to add an inorganic base in such an amount that it neutralizes the excess of mineral acid or sulfur dioxide, in addition to the above amount of the inorganic base.

The inorganic base used in the present invention may be of various form, for example, powdery or flaky. To promote the proceeding of neutralization of the mineral acid salt of amino acid alkyl ester effectively, powdery inorganic bases are preferable. Of these, inorganic bases having a specific surface area measured by a BET method of 0.8 m²/g or more, preferably 1.0 m²/g to 3.0 m²/g, are particularly preferable. When such an inorganic base having a large specific area is used, a neutralization reaction can proceed at almost the same speed as that when an aqueous solution of an inorganic base is used. The crystal lattice of the inorganic base is generally minute and the specific surface area thereof is generally about 0.5 m²/g or less in most cases. Therefore, when an inorganic base having a specific surface area of 0.8 m²/g or more is used, it is milled as required. Milling may be carried out by a known mill, such as a rod mill, ball mill, vibration mill, tower mill, colloid mill, jet mill or the like. Even when an inorganic base having a specific surface area of more than 3.0 m²/g is used, on the other hand, a technical advantage is no longer expected and it is hardly said to be economically advantageous to mill the inorganic base into fine powder using a mill or the like.

Any methods may be used without any restriction to bring a mineral acid salt of amino acid alkyl ester into contact with an inorganic base. The methods include, for example, one in which an inorganic base is directly added to an alcohol suspension of a mineral acid salt of amino acid alkyl ester, one in which an alcohol suspension of an inorganic base is added, and one in which an alcohol suspension of a mineral acid salt of amino acid alkyl ester is added to an alcohol suspension of an inorganic base. Further, when an alcohol having high solubility for alkali metal hydroxides, such as methanol, is used, an alkali metal hydroxide dissolved in the alcohol may be used.

Since a reaction between a mineral acid salt of amino acid alkyl ester and acrylonitrile is stoichiometrically an equimolar reaction in the present invention, acrylonitrile is used in an amount of at least 1 mol based on 1 mol of the amino group of the mineral acid salt of amino acid alkyl ester. However, when the amount is too large, a side reaction such as the polymerization reaction of acrylonitrile occurs. Therefore, the amount of acrylonitrile is generally 1 to 2 mol, preferably 1 to 1.6 mol, based on 1 mol of the amino group of the mineral acid salt of amino acid alkyl ester.

Any methods may be used without restriction to add acrylonitrile in the present invention. The methods include, for example, one in which acrylonitrile is added after a mineral acid salt of amino acid alkyl ester is brought into contact with an inorganic base and one in which acrylonitrile and an inorganic base are added simultaneously. Since an increase in the reaction scale causes an increases in the amount of neutralization heat between the inorganic base and the mineral acid, the method of adding acrylonitrile while observing the proceeding of a neutralization reaction after the injection of an inorganic base is preferable.

The concentration of the mineral acid salt of amino acid alkyl ester in the reaction medium is not particularly limited. However, when the concentration is too low, the yield per batch drops uneconomically, while when the concentration is too high, stirring or the like is adversely affected. The concentration is generally 0.1 to 60 wt%, preferably 1 to 50 wt%.

The temperature of the above reaction in the step (1) is not particularly limited. However, when the temperature is too low, the reaction rate significantly becomes low, while when the temperature is too high, a side reaction is promoted. Therefore, the temperature is preferably the freezing point to the boiling point of a system, more preferably 0 to 80°C. The reaction time cannot be given unconditionally because it greatly differs according to the type of mineral acid salt of amino acid alkyl ester but it is 1 to 30 hours, for example.

The reaction may be carried out under reduced pressure, atmospheric pressure or increased pressure.

The N-cyanoethyl amino acid alkyl ester formed by the reaction in the step (1) is reacted, without being isolated, with a dialkyl dicarbonate in the following step (2) to form an N-alkoxycarbonyl-N-cyanoethyl amino acid alkyl ester.

As for the type of dialkyl dicarbonate used in the present invention, any available dialkyl dicarbonates are used without restriction. Dialkyl dicarbonates whose two alkyl groups have 1 to 5 carbon atoms are preferable. The two alkyl groups may be the same or different and straight-chain or branched-chain. Of these, dialkyl dicarbonates whose two alkyl groups are the same are more preferable.

Illustrative examples of the dialkyl dicarbonate include dimethyl dicarbonate, diethyl dicarbonate, diisopropyl dicarbonate, di-tert-butyl dicarbonate, di-tert-amyl dicarbonate and the like. Of these, di-tert-butyl dicarbonate and di-tert-amyl dicarbonate which are stable against alcohols are particularly preferable.

The amount of the dialkyl dicarbonate used in the present invention is not particularly limited. However, when the amount is too small, the yield of the N-alkoxycarbonyl-N-cyanoethyl amino acid alkyl ester lowers, while when the amount is too large, the operation of separating an unreacted dialkyl dicarbonate from the reaction product is required. Therefore, the amount is preferably 0.9 to 1.2 mol, more preferably 0.9 to 1.1 mol, based on 1 mol of the N-cyanoethyl amino acid alkyl ester.

As for the temperature of a reaction between the N-cyanoethyl amino acid alkyl ester and the dialkyl dicarbonate in the present invention, when the temperature is too low, the reaction rate becomes low, while when the temperature is too high, the decomposition reaction of the dialkyl dicarbonate occurs. Therefore, the temperature is preferably the freezing point of the system to 60°C, more preferably 0 to 50°C. The reaction time is not particularly limited but it is preferably 0.1 to 10 hours. The reaction may be carried out under reduced pressure, atmospheric pressure or increased pressure.

The N-alkoxycarbonyl-N-cyanoethyl amino acid alkyl ester obtained in the present invention is isolated by the following method. That is, an alcohol is distilled off from the alcohol suspension as much as possible, water is added to dissolve the inorganic salt completely, the ester is extracted with an organic solvent from the obtained aqueous solution, and the organic solvent is distilled off to isolate the ester. Any organic solvents which are insoluble in water may be used without restriction as the organic solvent used for this purpose. Illustrative example of the organic solvent include esters such as ethyl acetate and propyl acetate; ethers such as diethyl ether and diisopropyl ether; aliphatic hydrocarbon halides such as methylene chloride, chloroform and carbon tetrachloride; aromatic hydrocarbons such as benzene, toluene and xylene; aromatic hydrocarbon halides such as chlorobenzene; aliphatic hydrocarbons such as hexane and heptane; ketones such as methyl isobutyl ketone; and carbonates such as dimethyl carbonate.

In order to remove the unreacted N-cyanoethyl amino acid alkyl ester and other impurities, the solution extracted with the organic solvent may be washed with an acidic aqueous solution and a basic aqueous solution. The acid used for this purpose is preferably a mineral acid such as hydrochloric acid, sulfuric acid, nitric acid or phosphoric acid, or organic acid such as acetic acid or citric acid. The base is preferably an alkali metal hydroxide such as sodium hydroxide or potassium hydroxide, or alkali metal carbonate such as sodium carbonate, potassium carbonate, sodium bicarbonate or potassium bicarbonate. The concentrations of the acidic aqueous solution and the basic aqueous solution are not particularly limited but are preferably 0.1 to 10 wt%.

The N-alkoxycarbonyl-N-cyanoethyl amino acid alkyl ester thus obtained can be used in the reaction of the following step directly or after purification. Any known purification methods may be used without restriction. For example, when the compound is liquid, it can be purified by distillation or the like, while when the compound is solid, it can be purified by recrystallization, reprecipitation or the like.

In the present invention, to produce the N-cyanoethyl amino acid alkyl ester by carrying out only the step (1), the N-cyanoethyl amino acid alkyl ester obtained in the above step (1) is isolated from the alcohol suspension of the compound at a high yield and purified.

Although any methods may be used without restriction to isolate and purify the N-cyanoethyl amino acid alkyl ester, the following method is effective. That is, after the end of a reaction between a mineral acid salt of amino acid alkyl ester and acrylonitrile, the deposited inorganic salt is separated by solid-liquid separation, and the compound is isolated by distilling off an alcohol from the obtained alcohol suspension and then purified by distillation or the like when the compound is liquid or by recrystallization, reprecipitation or the like when the compound is solid.

According to the present invention, an N-alkoxycarbonyl-N-cyanoethyl amino acid alkyl ester can be produced at a high yield by reacting a mineral acid salt of amino acid alkyl ester with acrylonitrile in an alcohol medium in the presence of an inorganic base to form an N-cyanoethyl amino acid alkyl ester and reacting the N-cyanoethyl amino acid alkyl ester obtained in the above step with a dialkyl dicarbonate. The N-cyanoethyl amino acid alkyl ester can also be produced at a high yield. Therefore, the present invention is industrially extremely useful as a process for producing an N-alkoxycarbonyl-N-cyanoethyl amino acid alkyl ester and an N-cyanoethyl amino acid alkyl ester.

The following examples are provided for the purpose of further illustrating the present invention but are in no way to be taken as limiting.

### Example 1

A hundred milliliters of ethanol and 27.92 g (0.2 mol) of glycine ethyl ester hydrochloride were injected into a 300-ml four-necked flask equipped with a stirrer and a thermometer, and 11.66 g (0.11 mol) of sodium carbonate was further added at room temperature while the above mixture was stirred. The resulting mixture was stirred for 30 minutes. Thereafter, 12.74 g (0.24 mol) of acrylonitrile was added, and the resulting mixture was allowed to react at 50 to 60°C for 6 hours to form N-cyanoethyl glycine ethyl ester. After cooling, 41.47 g (1.9 mol) of di-tert-butyl dicarbonate was added dropwise to the reaction suspension and stirred at room temperature for 4 hours.

After the end of the reaction, the reaction suspension was concentrated under reduced pressure, and 100 ml of water was added to the residue to extract a target substance with 100 ml of ethyl acetate. The organic layer was washed sequentially with 50 ml of 0.1 N hydrochloric acid, 50 ml of a 0.1 N aqueous solution of sodium bicarbonate and 50 ml of saturated brine, and then concentrated under reduced pressure to obtain 48 g of N-tert-butoxycarbonyl-N-cyanoethyl glycine ethyl ester. The yield was 93.8 %.

### Examples 2 to 12

The operation of Example 1 was repeated except that mineral acid salts of amino acid alkyl ester, alcohols and inorganic bases shown in Table 1 were used. The results are shown in Table 1.

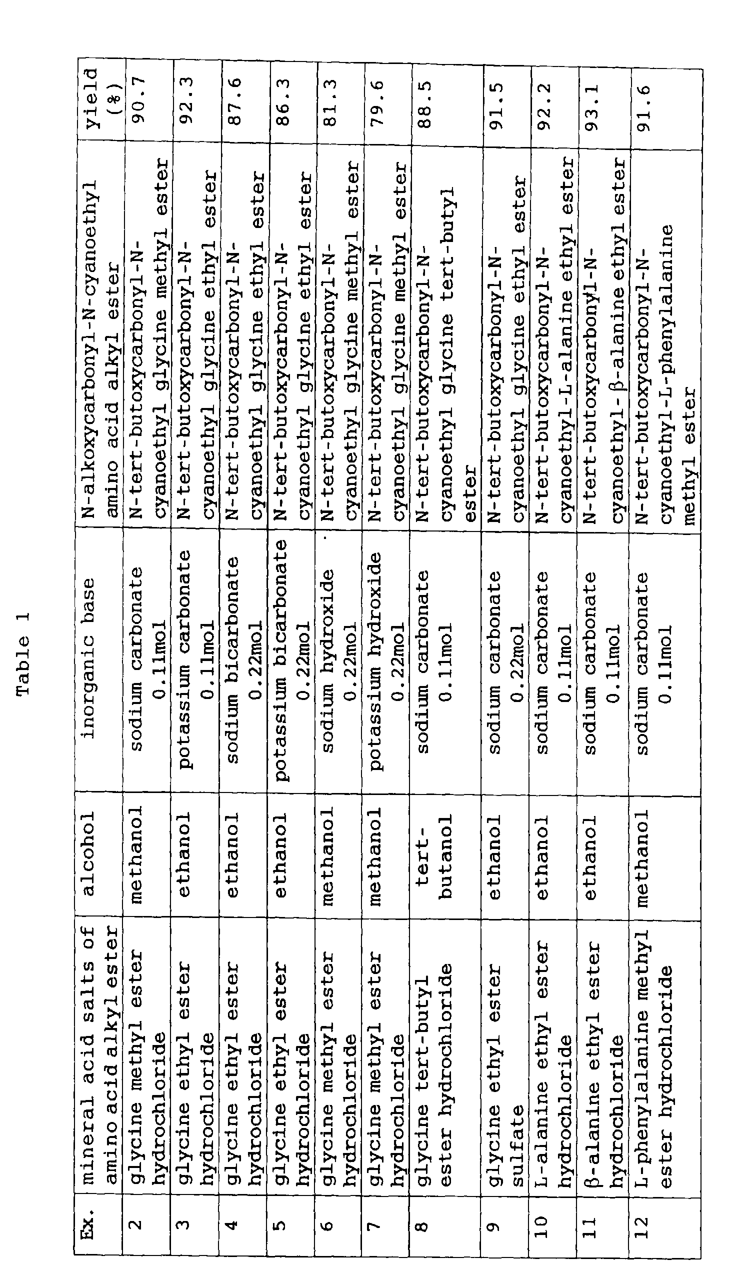

### Example 13

A hundred milliliters of ethanol was injected into a 300-ml four-necked flask equipped with a stirrer, a thermometer and a gas tube. After 14.6 g (0.4 mol) of hydrogen chloride was blown into the flask at 10°C or less under a nitrogen atmosphere, 15.01g (0.2 mol) of glycine was added, and the resulting mixture was stirred at 40°C for 6 hours to form glycine ethyl ester hydrochloride. After the end of the reaction, 22.26g (0.21 mol) of sodium carbonate was added at 10°C or less, and the resulting mixture was stirred for 30 minutes. Thereafter, 12.74 g (0.24 mol) of acrylonitrile was added, and the resulting mixture was allowed to react at 50 to 60°C for 6 hours to form N-cyanoethyl glycine ethyl ester. After cooling, 41.47g (1.9 mol) of di-tert-butyl dicarbonate was added dropwise to the reaction suspension, which was then stirred at room temperature for 4 hours.

After the end of the reaction, the reaction suspension was concentrated under reduced pressure, and 100 ml of water was added to the residue to extract a target substance with 100 ml of ethyl acetate. The organic layer was washed sequentially with 50 ml of 0.1 N hydrochloric acid, 50 ml of a 0.1 N aqueous solution of sodium bicarbonate and 50 ml of saturated brine, and then concentrated under reduced pressure to obtain 48.23 g of N-tert-butoxycarbonyl-N-cyanoethyl glycine ethyl ester. The yield was 94.2 %.

### Examples 14 to 20

The operation of Example 13 was repeated except that amino acids, alcohols and inorganic bases shown in Table 2 were used. The results are shown in Table 2.

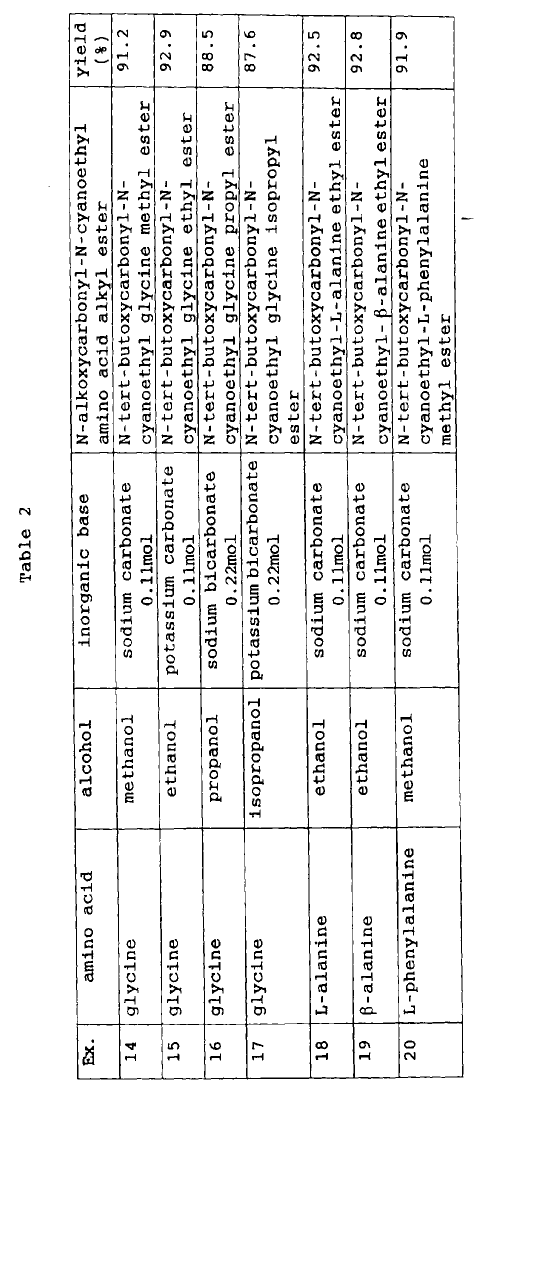

### Example 21

A hundred milliliters of ethanol and 27.92 g (0.2 mol) of glycine ethyl ester hydrochloride were injected into a 300-ml four-necked flask equipped with a stirrer and a thermometer, and 11.66 g (0.11 mol) of sodium carbonate was further added at room temperature while the above mixture was stirred. The resulting mixture was stirred for 30 minutes. Thereafter, 12.74 g (0.24 mol) of acrylonitrile was added, and the resulting mixture was allowed to react at 50 to 60°C for 6 hours.

After the end of the reaction, the reaction suspension was filtered, and the filtrate was concentrated under reduced pressure. Thereafter, the residue was distilled under reduced pressure to obtain 43.3 g of N-cyanoethyl glycine ethyl ester. The yield was 84.6 %.

### Examples 22 to 30

The operation of Example 21 was repeated except that mineral acid salts of amino acid alkyl ester, alcohols and inorganic bases shown in Table 3 were used. The results are shown in Table 3.

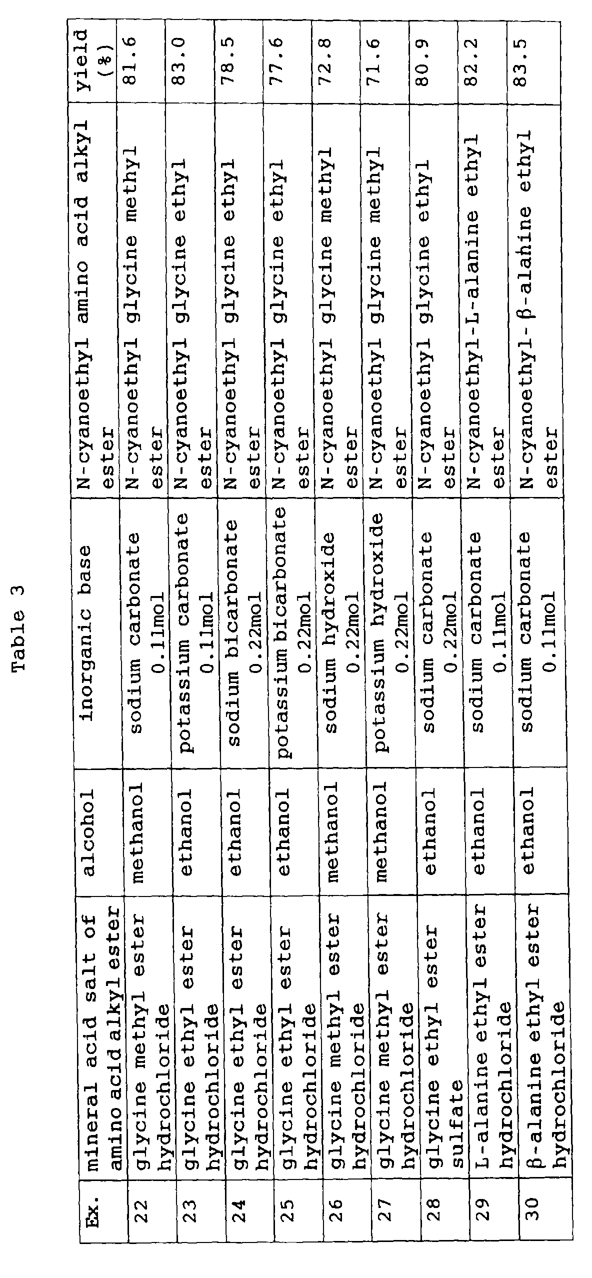

### Comparative Example 1

The operation of Example 21 was repeated except that toluene was used in place of ethanol. They yield of the obtained N-cyanoethyl glycine ethyl ester was 36.5 %.

### Comparative Example 2

The operation of Example 21 was repeated-except that tetrahydrofuran was used in place of ethanol. The yield of the obtained N-cyanoethyl glycine ethyl ester was 6.2 %.

### Comparative Example 3

The operation of Example 21 was repeated except that acetonitrile was used in place of ethanol. The yield of the obtained N-cyanoethyl glycine ethyl ester was 29.5 %.

## Claims

1. A process for producing an N-alkoxycarbonyl-N-cyanoethyl amino acid alkyl ester said process comprising the steps of:
(1) reacting a mineral acid salt of an amino acid alkyl ester with acrylonitrile in an alcohol medium in the presence of an inorganic base to form an N-cyanoethyl amino acid alkyl ester; and
(2) reacting the N-cyanoethyl amino acid alkyl ester with a dialkyl dicarbonate to form the N-alkoxycarbonyl-N-cyanoethyl amino acid alkyl ester.

2. The process according to claim 1, wherein the amino group of the amino acid alkyl ester is a primary amino group.

3. The process according to claims 1 or 2, wherein the alcohol medium is a straight-chain or branched-chain alcohol having 1 to 6 carbon atoms.

4. The process according to any one of the preceding claims, wherein the inorganic base is an alkali metal hydroxide, alkali metal carbonate, alkaline earth metal hydroxide or alkaline earth meal carbonate.

5. The process according to claim 4, wherein the inorganic base is a solid having a specific surface area measured by a BET method of 0.8 m²/g or more.

6. The process according to any one of the preceding claims, wherein acrylonitrile is used in an amount of 1 to 2 mol based on 1 mol of the amino group of the mineral acid salt of the amino acid alkyl ester.

7. The process according to any one of the preceding claims, wherein the reaction of step (1) is carried out at a temperature of from 0 to 80°C.

8. The process according to any one of the preceding claims, wherein step (1) is carried out and then, without isolating the N-cyanoethyl amino acid alkyl ester from the reaction mixture formed by step (1), step (2) is carried out.

9. The process according to any one of the preceding claims, wherein each of the two alkyl groups of the dialkyl dicarbonate independently has 1 to 5 carbon atoms.

10. The process according to any one of the preceding claims, wherein the dialkyl dicarbonate is used in an amount of 0.9 to 1.2 mol based on 1 mol of the N-cyanoethyl amino acid alkyl ester.

11. The process according to any one of the preceding claims, wherein the reaction of step (2) is carried out at a temperature of from 0 to 50°C.

12. A process for producing an N-cyanoethyl amino acid alkyl ester by reacting a mineral acid salt of an amino acid alkyl ester with acrylonitrile in an alcohol medium in the presence of an inorganic base.
